# EUROPEAN PATENT SPECIFICATION

(11) **EP 0 324 037 B1**
(45) Date of publication and mention of the grant of the patent: **03.09.1997**
(21) Application number: 88100233.1
(22) Date of filing: 11.01.1988
(51) Int. Cl.: A61K 49/00, G01N 33/74

(54) **Use of anabolic hormones for the manufacture of a medicament for the treatment of Alzheimer's disease and senile dementia**
Verwendung von Anabolika zur Herstellung eines Arzneimittels zur Behandlung von seniler Dementia und der Alzheimerschen Krankheit
Utilisation d'hormones anaboliques pour la fabrication d'un médicament pour le traitement de la demence sénile et de la maladie d'Alzheimer

(43) Date of publication of application: 19.07.1989
(73) Proprietor: Aroonsakul, Chaovanee, Chicago Illinois 60602 (US)
(72) Inventor: Aroonsakul, Chaovanee, Chicago Illinois 60602 (US)
(74) Representative: Gee, David William

(56) References cited:
- Arzneimittelforschung, 17(7), 1967, pp. 885-888
- Minerva Med., 60(8), 1967, pp. 315-319
- British Journal of Psychiatry, 150, 1987, pp. 674-681
- Chemical Abstracts, abstract no. 106:193998v
- Journal of Neurology, 233(6), 1986, pp. 370-372

## Description

### BACKGROUND OF THE INVENTION

The present invention is directed to the manufacture of a medicant for use in alleviating the symptoms of certain central nervous system diseases. These diseases for which the present invention has been found useful are: Alzheimer's disease; senile dementia. These have been found to affect different portions of the brain. Alzheimer's disease affects the portion of the brain vital to memory retention, the cortex. It is generally believed that the disease cuts off the cholinergic pathways by deficiencies of the enzyme choline acetyltranspherase, which is the chemical messenger from the nucleus basalis to the cortex. It has also been determined that in the above diseases, intrinsic depression develops, which is usually associated with an entirely different portion of the brain than those affected by the above-named diseases.

It is also known that in each of these diseases characterized by biochemical lesions in enzymes, membranes, or structure proteins of particular components, cellular atrophy is the result, which is a slow process of cellular deterioration, sometimes deferred bes, but leading to cellular atrophy, and eventually resulting in serious functional loss, causing the symptoms associated with each of the diseases. Such cellular atrophy generally occurs with advancing age, but, owing to these diseases, the processes are somehow speeded up, in a manner not known or understood at the present time. What is certain, however, is that in any cellular break-down, the enzymes required for protein synthesis are lacking. Further, it is also apparent that this lack of enzyme formation, and concomitant lack of protein synthesis, are caused by some interruption in the neural network through which biochemical signals are generated and transported. Thus, the very problem of each of these diseases runs to the basic structure of life: To wit, RNA and DNA, ribo nucleic acid and desoxy ribonucleic acid, which program each cell to provide the necessary enzymes for life-sustaining activity. Minerva Med., vol. 60, no.8, 1969, p.315-319 teaches that fluoxymesterone is indicated in presenility.
Brit. J. Psych., vol. 150, 1987, p.674-681 teaches that in Alzheimer-type dementia increased growth hormone plasma levels are observed.

Heretofore, there has been no effective means of treatment of each of the above-named diseases. In the case of Parkinson's disease, L-Dopa has been employed and has achieved some success. However, the period of efficacy of L-Dopa is limited to a few months, after which all of its beneficial effects disappear leaving only contraindication. In the case of Alzheimer's disease, and the remainder of the above-named diseases, ergoloid myselates have been used to some small success, but only in a very limited way, and have been successful only for a short time period. Further, ergoloid myselates suffer from the disadvantage of also causing unwanted side effects, that are often dangerous to the health and well-being of the patient. Just how this drug works is not known at this time, but it does offer some relief, though only temporary, to help restore mental acuteness, awareness and faculty.
The present invention is also directed to a method of diagnosing Alzheimer's disease in human beings.
Alzheimer's disease is the fourth leading cause of death in the United States, the precise cause of which is not yet known. At present, it is a difficult and time-consuming task to definitively determine whether a patient is suffering from Alzheimer's disease or another ailment having similar manifestations and/or symptoms. Presently-used techniques for determining Alzheimer's disease include neuropsychological testing which compares the mental status of the patient relative to a norm, as well as the patient's cognitive dysfunction. Such testing also tests for mood depressions, agitation, irritability, and the like, all of which are symptoms of Alzheimer's disease when combined with other diagnostic indications. These other diagnostic tools and methods are: The use of a brain atlas beam test or EEG (electroencephalogram) which demonstrate increases in delta and theta waves.
Further, CAT, computed axial tomograph, scans of the brain or nuclear magnetic resonance (NMR) are used in the diagnosis by showing reduced uptakes of the brain's cells in the temporal areas or at the hippocampal region, which is a sign of cortical atrophy, associated with Alzheimer's disease, since the cortex and hippocampus of the brain are those usually affected by Alzheimer's disease, such effect being caused, it is thought, by the deficiency of the enzyme choline acetyltransferase. Choline acetyltransferase is the chemical messenger from the nucleus basalis to the cortex. Still another method used in the diagnosis of Alzheimer's disease, based on the fact that it has been discovered that in Alzheimer's patients the brain is found to have consumed from 30%-50% less glucose in each of the four cortical regions and one subcortical region is the PET, positron emission tomograph, scan. The reduction of the consumption of glucose in these portions of the brain results, it is generally thought, from the deficiency of the enzyme phosphofructokinase, which is necessary for the conversion of glucose to high-energy intermediates. The determination of glucose reduction in these portions of the brain is typically carried out by the PET scan, which detects the presence of the glucose analogue -(F-18)-2-fluoro-2-deoxy-D-glucose (FDG).

It has hitherto been the conventional practice in order to make a diagnosis for Alzheimer's disease to utilize the positive indication of any of the above-mentioned tests. However, the results of these tests are often not definitive, since any one or more of them may show that the symptoms are not those of Alzheimer's disease even though the patient may be suffering therefrom. On the other hand, each of the above-mentioned tests may show that the diagnosis is that of Alzheimer's disease when, in fact, it is not. The frequency of these false positives may be high. For example, a false positive may occur if the patient is suffering from neurosyphylis, follic acid deficiency, Vitamin B12 deficiency, hypothyroidism, encephalitis, electrolyte imbalance, drug toxicity, other metabolic disorders, virus infection of central nervous system, cns, as well as others.

It is, of course, most important that a correct diagnosis be determined in order to decide upon the best treatment. The present invention is directed towards the incorporation to a novel diagnosis for senile dementia Alzheimer type Alzheimer's disease and senile dementia, that may be used in conjunction with other standard testing methods, or may be used alone for such determination, since this novel diagnosis has been found to be very accurate, especially when used in conjunction with conventional diagnostic tests for other chronic degenerative diseases of the cns form which similar indications may result, as to those of the method of the present invention, are not at all likely to affect the patient for whom Alzheimer's disease may be a likely diagnosis.

It has been known that the drug, levodopa (L-Dopa), a common drug for treating Parkinson's disease, causes increased secretion of the growth hormone somatotropin (HGH-human growth hormone). This finding of Aroonsakul and Allen provocative test (AA provocative test) such as L-Dopa's provocative stimulation of the pituitary gland to secrete HGH was used as the basis for viewing the functioning of the peripheral nervous system, (PNS), as an aminergic neuronetwork. Furthermore, since the hormone somatomedin-C (often referred to as IGF-I, for insulin-like growth factor, since it resembles insulin in many ways) is directly dependent upon the secretion of HGH by the pituitary gland, there has been established a direct linkage between increased secretion of HGH and increased production of the hormone somatomedin-C, which is produced chiefly in the liver and kidneys. The use of L-Dopa to markedly increase the secretions of somatotropin and, consequently, the production of somatomedin-C in the human body has led to what is generally termed the "L-Dopa provocative test". This test is used to determine the normal functioning of the anterior region of the pituitary gland responsible for the HGH production. Generally, the AA provocative test is used by detecting the increase of HGH and IGF-I in a blood serum by the use of radioimmunoassay (RIA), which determines the presence or absence or the amounts of a certain hormone in a serum by the use of a radioactive agent, used in vitro. Radioimmunoassay techniques were first discovered by the observation that unlabelled insulin displaces radioactive iodine-labelled insulin from insulin antibodies, in vitro. With the antibody concentration and radio-iodine-nated antigen held constant, the binding of the label is quantitatively related to the amount of unlabelled antigen that is added. Thus, any amount of an unlabelled antigen may be determined by the known standards therefor. The amount of unlabelled antigen in a serum-sample can be guaged by measuring the fraction of bound, labelled antigen in its presence from a standard. RIA involves separation of the labelled antigen that is of interest into bound-unbound fractions after the interaction with an antibody in the presence of the unknown quantity of unlabelled antigen to be measured. RIA has been used in the past for measuring the amount of HGH in a serum taken from a subgroup of children, for treating diseases in children related to growth. The radioactive element used in this determination is usually I-125.

Somatotropin is a growth hormone (polypeptic-link amino acid in character), secreted by the anterior region of the pituitary gland, which is most known for its capability of causing growth of the human skeleton. This is also known as the human growth hormone (HGH), and is the precursor of the hormone somatomedin-C (IGF-I), produced by the liver and kidneys. According to the present invention, it has been discovered that patients suffering from Alzheimer's disease have a deficiency of somatotropin production which leads to a deficiency of somatomedin-C levels, and that exogenous stimulation by a drug to cause increased secretions of HGH in normal human subjects, does not function normally in Alzheimer's patients. Though it has been known to have increased levels of IGF-I in the blood with reduced levels of HGH, these instances are rare and can be taken into consideration when determining the diagnosis according to the present invention.

### SUMMARY OF THE INVENTION

The present invention consists of the use of an anabolic sex hormone selected from oxymetholone, oxandrolone, ethylestenol, stanozolol, nandrolone phenpropionate, nandrolone decanoate, and methandriol for the manufacture of a medicant for use in alleviating the symptoms of one of Alzheimer's disease and Senile Dementia.

For male patients, preferably the medicant additionally contains testosterone. For female patients, preferably the medicant additionally contains at least one of a human growth hormone selected from the group somatotropin and chorionic gonadotropin, and an anabolic sex hormone selected from the group estradiol, estrone, estriol and conjugated estrogen.

For both male and female patients, additional drugs may be added to ensure that the induction of hormone into the blood stream is effected, so that the medicant is delivered to the site of the brain causing the disfunction and symptoms. Thus nonsteroidal anti-inflammatory drugs and vasodilators may be used in combination with the hormone, to ensure that the blood delivers the hormone or hormones to the brain. Further, the medicant may additionally contain a human growth hormone, which are known for their anabolic tendencies, especially in those cases where there has been detected a noticeable loss of such growth hormone in the patient's system, which growth hormones also exhibit remarkable rejuvenating properties.

It is, therefore, a primary objective of the present invention to provide a diagnostic technique by which positive indication of Alzheimer's disease in a human being may be established definitively.

It is another objective of the present invention to provide a novel diagnostic method to be used in conjunction with presently-available and presently-used techniques for the determination of Alzheimer's disease. It is yet another objective of the present invention to provide another technique for diagnosing Alzheimer's disease in human beings that is easily performed by conventional radioimmunoassay techniques.

It is still another objective of the present invention to provide a method of diagnosing Alzheimer's disease and senile dementia in human beings which includes the steps of: {a} determining the level of somatotropin in at least one blood serum sample taken from a patient following somatotropin stimulation; {b} comparing the said level with a known standard for blood serum samples taken from patients following somatotropin stimulation; and {c} noting any reduced increase in the level of somatotropin as an indicator of at least one of Alzheimer's disease and Senile Dementia for the said patient.

According to the method of diagnosing Alzheimer's disease of the present invention, the patient suspected of suffering from this disease is subjected to the L-Dopa AA provocative test, by which a specified dosage is administered on a first day, and followed on the next day with a higher dosage, immediately after which blood-serum samples are taken at 30 minute intervals, up to 120 minutes after the initial administration of the L-Dopa on the second day, for subsequent analysis by RIA techniques in order to determine the absolute and relative levels of somatotropin and somatomedin-C hormones in each sample, for subsequent comparison with levels of normal persons in the same age bracket. By comparing the test-sample levels with those of normal persons, a firm diagnosis of Alzheimer's disease may be made, if the increase of these hormones fall below a critical value established by statistical techniques based on normal subjects.

### BRIEF DESCRIPTION OF THE DRAWING

The invention will be more readily understood with reference to the accompanying drawing, wherein:
Figure 1 is a bar-chart showing the levels of the hormone somatotropin in blood sera at the end of 30 minutes, 60 minutes, 90 minutes and 120 minutes, as well as at the time of initial administration, obtained from the results of the L-Dopa AA provocative test, for determining levels thereof relative to a statistical average of normal subjects according to the method of the present invention for diagnosing Alzheimer's disease in human beings;
Figure 2 is a bar-chart similar to Figure 1 showing the same information for the hormone somatomedin-C;
Figure 3 is graph showing the comparison between the human growth hormone levels of a group of normal subjects as well as the graphs for those diagnosed as suffering from Alzheimer's disease and senile dementia; and other central nervous system disorders; and
Figure 4 is a graph showing the levels of brain neuro-transmitter, cholineacethylesterase, present in blood samples according to the method of diagnosing of the present invention for a group of normal control subjects, patients suffering from Alzheimer's disease, and patients suffering from senile dementia.

### DETAILED DESCRIPTION OF THE INVENTION

To reverse the degenerative nature of Senile Dementia and Alzheimer's disease it has been discovered that treatment methods utilizing the synthesizing, metabolic effects of androgens, estrogens, and anabolic hormones have reversed the degenerative nature of the diseases, and have restored patients suffering from the diseases to more normal and productive lives, with the alleviation of many of the symptoms of the diseases. Further, upon continual and prolonged treatment with the above-named group of hormones, there has not been found any diminution of efficacy of the treatment, nor any serious contraindications and adverse side effects that would tend to discourage such treatment.

In the use according to claim 1 it has been found that treating patients with the claimed hormones has caused alleviation of the degenerative nature of the diseases, and has resulted in putting the patient back on the road toward normalcy.

Further, combinations of anabolic hormones alone, or anabolic hormones with androgens, may also be used to find the precisely-desired anabolic effect to be had on the central nervous system of the patient, again with the minimum daily dosage of 1 mg, either for combinations, or individually-administered hormones.

Patients who are in the late and/or vegetative stages need other medication. For women, estradiol, a major anabolic sex hormone in a female is needed. For men, androgen, a major anabolic sex hormone in a male is needed. Thus, in one case history of a female patient 78 years of age, weighing approximately one-hundred fifty pounds, diagnosed as having Alzheimer's disease, the patient was given the following, orally: 1.25 mg conjugated estrogen once a day; 10 mg. methyltetosterone once a day; 1 mg ergoloid myselate USP four times a day; 50 mg dipyridamole four times a day; and 300 mg acetyl salicylic acid enteric coated four times day.

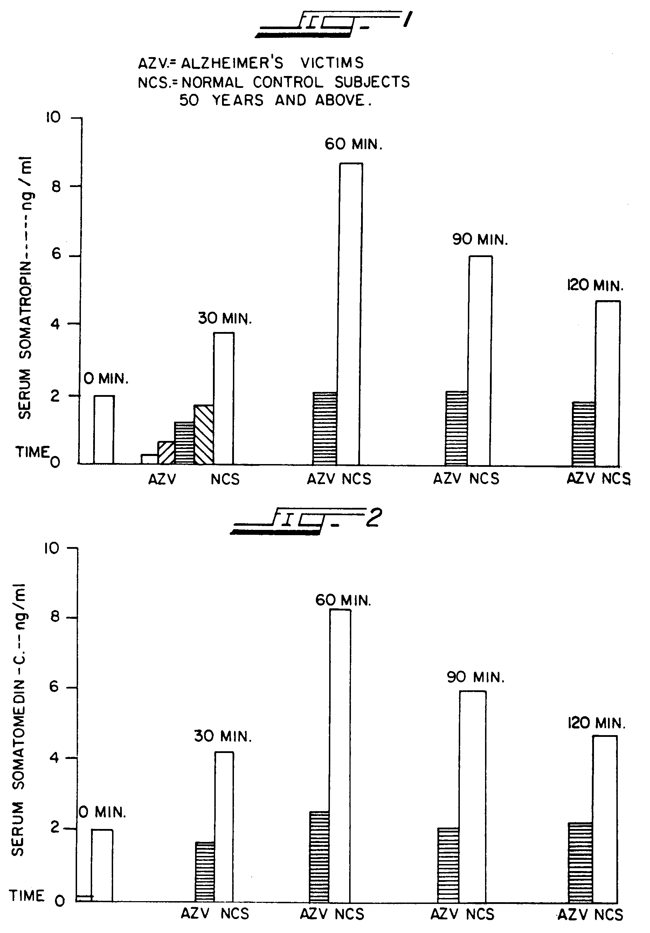

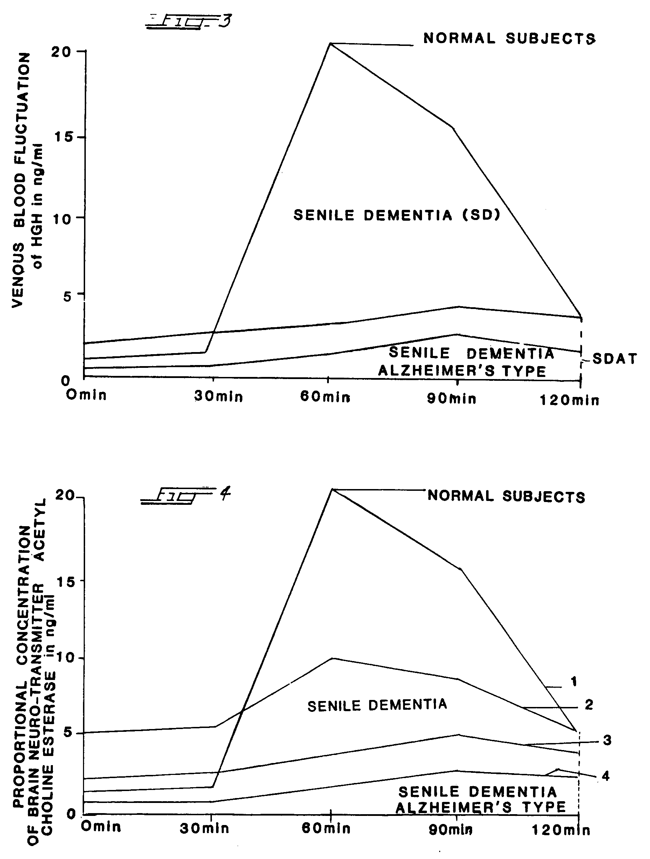

The ergolid myselate helps, it is thought in the art, that it will serve to prevent the cell's temporary loss of protein; the dipyridamole increase cerebral blood flow; and the acetyl salicylic acid helps to prevent clots among other beneficial results. Just as in the case of the male patient noted above, this female patient experienced marked and fast rejuvenation, dissipation of dementia, increased mental alertness, and a general vitalization such that many of her Alzheimer disease symptoms by conventional diagnosis; but senile dementia disappeared by new biochemical diagnosis in figure 3 and 4.

Besides the use of conjugated estrogen, any estrogen from the following group may be used singly or in combination: Estradiol; estrone; estriol. Further, gonadotropins and chorionic gonadotropins may also be used, singly or in combination. Also, another androgen or an anabolic hormone could have been used successfully instead of the methyltestosterone.

In conjunction with the above-disclosed medicant , in these patients where it has been determined that the level of growth hormone in their system is below normal for their size, sex, and age bracket, the above drugs are supplemented with a growth hormone to reestablish normal levels thereof in the patients. These growth hormones, also of potent anabolic efficacy, serve to restore body function to a state receptive to the medicant of the present invention in those cases where growth hormone levels are deficient. Further, the growth hormone may also be used in the medicant for those patients having normal levels of old age thereof, if it has been found that the androgen or anabolic hormones alone or in combination have not adequately provided success. Typically, if after six months treatment with the medicant of the present invention, improvement has not been shown, administering of growth hormones should be instituted, e.g. chorionic gonodotropin in combination. In the advanced stages of the diseases, a growth hormone is also used at the outset of treatment, since owing to its increased and magnified positive metabolic effect, a greater and more concentrated inducement of nerve cell regeneration is needed as described in the diagnostic method of senile dementia Alzheimer style.

It is also indicated that the medicant treatment as described in the present invention is successful for the alleviating of at least some symptoms for most patients in the incipient, beginning or intermediate stages of the above-named diseases because of the following:
1. General increase in cerebral blood flow which enhances oxidation, including the normal metabolism of the brain cells;
2. Decrease of catabolism of protein and amino acids;
3. Enhancement of protein anabolism, leading to increased activity of brain cells, with the concomitant increase in red blood cell production;
4. Increase in the retention of calcium and sodium, which improve the axon-presynaptic-postsynaptic cell transmission;
5. Increase of intercellular protein, which increases the formation of DNA andd RNA;
6. The revitalization of the nerve cell body dendrite and axons of the pre-synaptic and post-synaptic cells.

It is, of course, to be understood that the dosage of the medicant of the present invention given is dependent upon the weight, size age, and the like of the patient being treated. Further, any other well-known and equivalent sex hormone and anabolic hormone may be used in addition to those listed above, as long as the anabolic manifestations thereof are prevalent. Though the use of sex hormones, growth hormones, and anabolic hormones have been suspected of causing carcinoma, however, that in the doses above-stated for use in the treatment of the present invention, such likelihood is not of great probability. (Suspected cancer patients will be excluded from the treatment.) further, since many of the patients for whom the present treatment would be useful and for whom it would prove successful, are quite advanced in age, and since their conditions are at present hopeless and their lives, for all intents and purposes, less than totally productive, it is definitely shown that the beneficial and successful attributes of the treatment of the present invention far outweigh the possible negative side effects and hazards.

In order to properly diagnose Alzheimer's disease, it has been discovered that patients suffering from this disease show a depletion of the human growth hormone somatotropin (HGH) and the HGH-dependent hormone somatomedin-C (IGH-I) which makes connective tissue, die on skin and joints. The growth hormone is secreted by the pituitary gland, while somatomedin-C is secreted mainly by the liver and kidneys by stimulation of somatotropin. This discovery of the lack of proper levels of these two hormones, in combination with the fact that Alzheimer's patients lack the capabilities of producing these hormones endogenously even when exogenous stimuli are created in the body of the patient, has led to the method of diagnosis of the present invention. According to the present invention, the L-Dopa provocative test of Aroonsakul and Allen is used to cause increased secretions of these two hormones, or somatropin alone, in order to determine if the pituitary gland, and the liver and kidneys, are capable of producing these hormones in response to this provocative test. It is important that not only the absolute levels of these two hormones in the peripheral blood serum be tested before the L-Dopa provocative test of Aroonsakul and Allen but also the levels of the increase, if any, produced by the L-Dopa provocative test, in order to determine the current stimulation-capabilities of the peripheral nervous system and the hypothalamus, as well as the beta-adrenergic, alph adrenergic, and dopaminergic control systems. It is believed that the dominant control system is the dopaminergic, which is why L-Dopa causes increased secretions of HGH. For example clonidine propenolol, serotonin, ergoloid myselate causes andrenergic stimulation of the brain which will increase HGH.

The secretion of somatotropin by the pituitary gland is dependant upon many factors, some of them being well-known, such as physical exercise, physical and hypoglycemic stresses, low-protein intake and others. Endogenous triggers of HGH release include sleep. Whether exogenous or endogenous, the changes in the secretion of the growth hormone (somatotropin) by the pituitary gland are directly dependent upon by the hormone "growth-hormone releasing-hormone" (GHRH) produced by the hypothalamus, which acts directly upon the pituitary gland in order to cause increase secretion of HGH. Exogenous injection of GHRH into the blood system will result in direct increased secretion of HGH. The secretion of HGH, in turn, causes the increased secretion of somatomedin-C by the liver and kidneys, which somatomedin-C is also used as a feedback loop to the hypothalamus to regulate the secretion of GHRH and, thus, the secretion of HGH. The secretion of HGH is also inhibited by the hormone somatostatin, a cyclic peptide having fourteen amino acids, produced by the pancreas. Somatostatin also acts in a feedback loop with the hypothalamus to inhibit production of GHRH and HGH.

It has been the discovery, according to the present invention that patients suffering from Alzheimer's disease lack proper and normal levels of HGH and somatomedin-C, and also lack the capabilities of producing increased amounts of these hormones in response to exogenous stimuli that tend to cause substantially large and immediate increases of secretions in these two hormones, such as occurs during the Aroonsokul-Allen provocative test (dopomine, catecholamine, serotonin). It is the method of the present invention to use the L-Dopa provocative test to cause sudden and substantial increases in the secretions of the hormones HGH and somatomedin-C. By using radioimmunoassay techniques for detecting the levels of these hormones in the blood serum at chosen time intervals, the results therof may be compared with the norm for the age of the patient, to thereby guage if the pituitary gland is capable of reacting to this stimuli to increase production of HGH, so that a firm diagnosis of Alzheimer's disease may be made.

According to the method of the present invention for diagnosing Alzheimer's disease, one day prior to the use of the Aroonsokul-Allen provocative test provocative test, a dosage of 8mg. og L-dopa per kilogram-of-weight of the patient is administered to the patient in order to activate the HGH-secreting capabilities of the pituitary gland. On the next day, after an overnight fast, the L-dopa provocative test is given, with a dosage of 15mg. of -dopa per kilogram-of-weight of the patient. A blood sample is taken just prior to the dosage of 8mg. of L-dopa per kg. on the day prior to the L-Dopa provocative test, and blood samples are also taken immedidately before the start of the L-Dopa provocative testing, and every 30 minutes after the dosage of 15mg./kg. has been administered. Each blood serum is then tested by RIA techniques to determine the absolute levels of somatotropin and somatomedin-C, and compared against the normal values for the age of the patient being tested. Referring to Figure 1, there is shown a bar-chart comparing the levels of somatotropin and somatomedin-C obtained by RIA techniques for the five blood sera taken during the provocative test with the levels for normal subjects aged fifty and over. The shaded bars represent typical levels of a patient who has Alzheimer's disease, while the unshaded bars indicate the levels for a normal person aged fifty and over, for the five sera assayed. As it may be seen, for normal subjects, the readings at times: 0 minutes, 30 minutes, 60 minutes, 90 minutes, and 120 minutes are, approximately: 2.00 ng/ml; 3.75 ng/ml; 7.90 ng/ml; 6.05 ng/ml; and 4.45 ng/ml. For patients suffering from Alzheimer's disease the corresponding values are, approximately: 0.02 ng/ml; between 0.03 ng/ml and 1.85 ng/ml; 2.15 ng/ml; 2.05 ng/ml; and 1.85 ng/ml. The values indicated for the normal subjects are the statistical mean average. The highest value shown in figure 1, for a patient suffering from Alzheimer's disease, for each of the sera tested by RIA is that lying outside of the standard statistical error associated with the RIA testing.

Figure 2 shows similar results for the RIA of the hormone somatomedin-C. For normal subjects age 50 and over, the values from RIA of this hormone are appproximately: 1.95 ng/ml; 4.00 ng/ml; 8.05 ng/ml; 5.90 ng/ml; and 4.45 mg/ml. The corresponding values of Alzheimer's disease patients, are approximately: 0.10 ng/ml; 1.65 ng/ml; 2.15 ng/ml; 2.00 ng/ml; and 2.05 ng/ml.

It is, of course, possible to use different time periods in which the blood sera are taken for subsequent RIA analysis, with these results being compared to a standard for subjects aged 50 and over for the same time periods tested. According to the present invention, substantial differences in levels of these two hormones during the AA provocative test as compared with the normal subjects is a positive and definitive indication of Alzheimer's diseasse.Furthermore, the absolute differences between the tested sera and the levels for normal subjects may also be used for an indication as to the state of advancement of Alzheimer's disease in the patient. Tested levels far outside the statistical norm adjusted for standard statistical error would mean a more advanced stage of the disease.

There will be some instances where the levels of somatotropin will increase and be similar to those of normal subjects, but the levels of somatomedin-C will fall far short of those for normal subjects, as could occur if the patient were suffering from liver disease. Thus, the AA provocative test would show a mixed result. In this case, further testing would be required, and a positive determination of Alzheimer's disease would have to be confirmed in conjunction with other, currently-used, prior art methods of diagnosis, such as EEG testing, neuropsychological testing, and the like. Also, where the results from the AA provocate test do not show enough differences between the patient being tested and the norm, such as might occur if the values determined by RIA were not outside the statistical mean error of the normal group, then these other conventional methods of diagnosis would be used in conjunction with the method of the present invention. Whereas, both HGH and IGF-I deficiency in children may occur, as in dwarfism, such matched deficiency in adults is not known to indicate any other disease but that discovered according to the present invention. Since during basal, morning conditions there usually cannot be detected any differences between normal subjects and those with HGH deficiency, the provocative test is required, as described above.

Any other HGH-provocative agent may be used instead of L-Dopa. For example, bromocriptine, propanolol, serotonin,catecholamine clonidine, other dopaminergic stimuli, and glucagon, a small peptide that mediates the flow of glucose to insulin-independent tissues. Of course, the time periods between which the blood sera are taken will depend upon the somatotropin-provocative agent used. Other constraints and conditions will, of course, change dependent upon the HGH-provocative used, and will be obvious to one having ordinary skill in the art. For example, for glucagon as the HGH-provocative, overnight fasting would not be required.

The same type of provocative test above described may also be used for diagnosing senile dementia in human beings, as shown in figures 3 and 4, which are graphs of the levels of the hormone being detected for the subject patient diagnosed as suffering from Alzheimer's disease or senile dementia as compared to a controlled group thereof. The same method of diagnosing is carried out as above described when using the desired provocative test. It has also been discovered that the detection of the proportion of the brain neuro-transmitter, cholineacethylesterase, is also indicative of a diagnosis of Alzheimer's disease or senile dementia, as shown in the graphs of Figure 4 where the ordinate is time and the abcissa is the proportion of the brain neuro-transmitter, acetylcholineesterase, to the total blood sample for normal subjects and those suffering from Alzheimer's disease or senile dementia. In Figure 4, the graph labelled 1 shows the readings of the proportion of the bain transmitter acetlycholineestrase for normal subjects, while the graph labelled 2 shows the upward limit of the readings for the proportion of the brain transmitter indicative of Senile Dementia. The graph labelled 4 indicates the readings at or below which is an indication of Alzheimer's Disease.The region between the graphs labelled 3 and 4 is a transition region, readings lying therein not being clearly definitive as to Senile Dementia or Alzheimer's Disease, though at least one is present.

## Claims

1. Use of an anabolic sex hormone selected from oxymetholone, oxandrolone, ethylestenol, stanozolol, nandrolone phenpropionate, nandrolone decanoate, and methandriol for the manufacture of a medicant for use in alleviating the symptoms of one of Alzheimer's disease and Senile Dementia.

2. Use according to Claim 1 wherein the medicant additionally contains a human growth hormone.

3. Use according to Claim 2 wherein the human growth hormone is chorionic gonadotropin.

4. Use according to claim 1 for a male patient wherein the medicant additionally contains testtosterone.

5. Use according to Claim 1 for a female patient wherein the medicant additionally contains at least one of a human growth hormone selected from the group somatotropin and chorionic gonadotropin, and an anabolic sex hormone selected from the group estradiol, estrone, estriol and conjugated estrogen.

6. A method of diagnosing Alzheimer's disease and Senile Dementia in human beings which includes the steps of
{a} determining the level of somatotropin in at least one blood serum sample taken from a patient following somatotropin stimulation,
{b} comparing the said level with a known standard for blood serum samples taken from patients following somatotropin stimulation, and
{c} noting any reduced increase in the level of somatotropin as an indicator of at least one of Alzheimer's disease and Senile Dementia for the said patient.

7. A method according to Claim 6 characterised in that the level of somatomedin-C is determined in said at least one sample, and in that the known standard is derived from said blood serum samples from normal subjects within the same age group.

## Patentansprüche

1. Verwendung eines aus Oxymetholon, Oxandrolon, Ethylestenol, Stanozolol, Nandrolon-Phenpropionat, Nandrolon-Decanoat und Methandriol selektierten anabolischen Sexualhormons für die Herstellung eines Medikaments zur Verwendung bei der Linderung der Symptome von entweder Alzheimerischen Krankheit oder Altersdemenz.

2. Verwendung nach Anspruch 1, worin das Medikament zusätzlich ein menschliches Wachstumshormon enthält.

3. Verwendung nach Anspruch 3, worin das menschliche Wachstumshormon Choriongonadotropin ist.

4. Verwendung nach Anspruch 1 für einen Patienten, worin das Medikament zusätzlich Testosteron enthält.

5. Verwendung nach Anspruch 1 für eine Patientin, worin das Medikament zusätzlich mindestens eins eines menschlichen Wachstumshormon enthält, das aus der Gruppe Somatotropin und Choriongonadotropin ausgewählt ist, und ein anabolisches Sexualhormon, das aus der Gruppe Östradiol, Östron, Östriol und konjugiertem Östrogen ausgewählt ist.

6. Eine Methode des Diagnostizierens der Alzheimerischen Krankheit und von Altersdemenz in Menschen, die Schritte folgende Schritte einschließt:
(a) Bestimmen des Niveaus von Somatotropin in mindestens einer Blutserumprobe, die einem Patienten im Anschluß an Stimulation mit Somatotropin entnommen wurde,
(b) Verleichen des genannten Niveaus mit einer bekannten Norm für Blutserumproben, die Patienten im Anschluß an Stimulation mit Somatotropin entnommen wurden, und
(c) Notieren jeder reduzierten Zunahme im Niveau von Somatotropin als entweder Indikator von mindestens einer Alzheimerischen Krankheit oder Altersdemenz oder beide für den genannten Patienten.

7. Eine Methode nach Anspruch 6, dadurch gekennzeichnet, daß das Niveau von Somatomedin-C in genannter mindestens einer Probe bestimmt wird und, dadurch, daß die bekannte Norm ab genannten Blutserumproben ab normalen Versuchspersonen innerhalb derselben Altersgruppe stammt.

## Revendications

1. Emploi d'une hormone anabolique sexuelle sélectionnée à partir d'oxymétholone, d'oxandrolone, d'éthylesténol, de stanozolol, de nandrolone phenpropionate, de nandrolone décanoate et de méthandriol pour la fabrication d'un médicament à utiliser pour soulager les symptômes d'une maladie d'Alzheimer on de démence sénile.

2. Emploi selon la revendication 1, dans lequel le médicament contient en plus une hormone humaine de croissance.

3. Emploi selon la revendication 2, dans lequel l'hormone humaine de croissance est de la gonadotrophine chorionique.

4. Emploi selon la revendication 1 chez un patient mâle, dans lequel le médicament contient en plus de la testostérone.

5. Emploi selon la revendication 1 chez une patiente femelle, dans lequel le médicament contient en plus au moins une hormone humaine de croissance sélectionnée à partir du groupe somatotrophine et gonadotrophine chorionique et une hormone anabolique sexuelle sélectionnée à partir du groupe oestradiol, oestrone, oestriol et oestrogène conjugué.

6. Une méthode pour diagnostiquer la maladie d'Alzheimer et la démence sénile chez les êtres humains qui inclut les étapes consistant à :
(a) déterminer le niveau de somatotrophine dans au moins un échantillon de sérum sanguin prélevé chez un patient suite à une stimulation de la somatotrophine,
(b) comparer ledit niveau avec un étalon connu pour des échantillons de sérum sanguin prélevés chez des patients suite à une stimulation de la somatotrophine, et
(c) noter toute augmentation réduite du niveau de somatotrophine comme un indicateur d'une maladie au moins d'Alzheimer on de démence sénile chez ledit patient.

7. Une méthode selon la revendication 6 caractérisée en ce que le niveau de somatomédine C est déterminé dans ledit au moins un échantillon et en ce que l'étalon connu provient desdits échantillons de sérum sanguin de sujets normaux appartenant au même groupe d'âge.
